(19) Europäisches Patentamt
European Patent Office
Office européen des brevets

(11) Veröffentlichungsnummer: **0 072 438**
**A2**

(12) # EUROPÄISCHE PATENTANMELDUNG

(21) Anmeldenummer: 82106389.8

(22) Anmeldetag: 16.07.82

(51) Int. Cl.³: C 07 C 127/22
C 07 D 295/12, C 07 D 209/02
C 07 D 211/14, C 07 D 209/08
C 07 D 265/30, A 01 N 47/34

(30) Priorität: 13.08.81 DE 3132020

(43) Veröffentlichungstag der Anmeldung: 23.02.83 Patentblatt 83/8

(84) Benannte Vertragsstaaten: BE CH DE FR GB IT LI NL

(71) Anmelder: BASF Aktiengesellschaft
Carl-Bosch-Strasse 38
D-6700 Ludwigshafen(DE)

(72) Erfinder: Lange, Arno, Dr.
Friedrichsplatz 11
D-6800 Mannheim(DE)

(72) Erfinder: Kiehs, Karl, Dr.
Sudetenstrasse 22
D-6840 Lampertheim(DE)

(72) Erfinder: Adolphi, Heinrich, Dr.
Kalmitweg 11
D-6703 Limburgerhof(DE)

(54) **N-Benzoyl-N'-phenylharnstoffe, Verfahren zu ihrer Herstellung und ihre Verwendung zur Bekämpfung von Insekten und Spinnentieren.**

(57) N-Benzoyl-N'-phenylharnstoffe der Formel

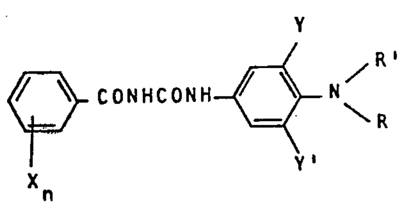

(1),

in der X, n, Y, Y', R und R' die in der Beschreibung genannten Bedeutungen haben, Verfahren zu ihrer Herstellung und ihre Verwendung zur Bekämpfung von Insekten und Spinnentieren.

EP 0 072 438 A2

Croydon Printing Company Ltd

N-Benzoyl-N'-phenylharnstoffe, Verfahren zu ihrer Herstellung und ihre Verwendung zur Bekämpfung von Insekten und Spinnentieren

Die vorliegende Erfindung betrifft N-Benzoyl-N'-phenyl-harnstöffe, Verfahren zu ihrer Herstellung und insektizide und akarizide Mittel, die diese Verbindungen als Wirkstoffe enthalten.

Es ist bekannt, daß N-Benzoyl-N'-(4-dialkylaminophenyl)-harnstoffe (J. Agr. Food. Chem. 21, 353 (1973)) und N-Benzoyl-N'-phenylharnstoffe (EP-OS 16729) insektizid wirksam sind.

Es wurde gefunden, daß N-Benzoyl-N'-phenylharnstoffe der Formel

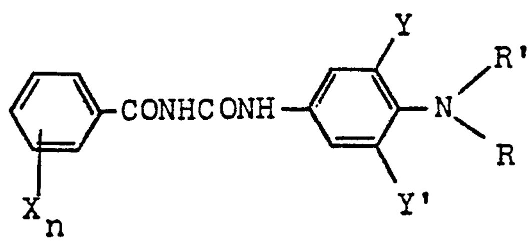 (I),

in der
X Chlor, Fluor, Brom oder Methyl,
n 0, 1, 2 oder 3,
Y und Y' unabhängig voneinander Wasserstoff, Fluor, Chlor, Brom, Methyl, Trifluormethyl und
R und R' unabhängig voneinander einen gegebenenfalls substituierten aliphatischen Kohlenwasserstoffrest mit 1 bis 15 Kohlenstoffatomen, wobei R und R' nicht gleichzeitig Methyl sein können, oder einen gegebenenfalls substituierten Phenylrest bedeuten oder zusammen einen gegebenenfalls durch Alkyl oder Phenyl einfach oder zweifach substituierte Alkylenkette mit 4 oder 5 Kohlenstoffatomen, die durch Sauerstoff unterbrochen sein kann, bilden oder zusammen mit

Mu/Kl

dem Stickstoffatom, dessen Substituenten R und R' sind, einen gegebenenfalls durch Methyl einfach oder mehrfach substituierten Octahydroindolyl- oder Methanoazepanylrest bilden, und R' auch Wasserstoff oder Acetyl bedeutet, wenn R für einen substituierten Phenylrest steht, mit der Maßgabe, daß R und R' eine gegebenenfalls durch Alkyl oder Phenyl einfach oder zweifach substituierte Alkylenkette mit 4 oder 5 Kohlenstoffatomen, die durch Sauerstoff unterbrochen sein kann, bilden oder zusammen mit dem Stickstoffatom, dessen Substituenten R und R' sind, einen gegebenenfalls durch Methyl einfach oder mehrfach substituierten Octahydroindolyl- oder Methanoazepanylrest bilden, wenn Y und Y' jeweils Chlor oder Brom bedeuten, insektizid und akarizid wirksam sind.

Aliphatische Kohlenwasserstoffreste für R und R' in Formel I sind unverzweigte oder verzweigte Alkyl-, Alkenyl- oder Alkinylreste mit bis zu 15 Kohlenstoffatomen, vorzugsweise 1 bis 12, insbesondere 1 bis 9, Kohlenstoffatomen, die durch Phenyl substituiert sein können, wie Methyl, Ethyl, n-Propyl, i-Propyl, tert.-Butyl, i-Butyl, sec-Butyl, n-Pentyl, i-Pentyl, n-Hexyl, n-Octyl, n-Nonyl, n-Dodecyl, n-Pentadecyl, Benzyl, Phenethyl, Allyl, Methallyl, Crotonyl, Propargyl, But-1-inyl, But-2-inyl, Cycloalkylreste mit 3 bis 6 Kohlenstoffatomen, wie Cyclopentyl, Cyclohexyl, Cyclobutyl, Cyclooctyl. R und R' können dabei nicht gleichzeitig für Methyl stehen.

Substituierte Phenylreste für R und R' in Formel I sind beispielsweise durch Halogen, wie Chlor oder Brom substituierte Phenylreste, wie 4-Chlorphenyl, 4-Bromphenyl, 2,4-Dibromphenyl, Phenyl.

R und R' in Formel I können zusammen eine gegebenenfalls durch Alkyl mit 1 bis 4 Kohlenstoffatomen, vorzugsweise Methyl oder Ethyl, oder Phenyl substituierte Alkylenkette mit 4 oder 5 Kohlenstoffatomen, die durch Sauerstoff unterbrochen sein kann, bilden, so daß zusammen mit dem Stickstoffatom, dessen Substituenten R und R' sind, Pyrrolidin-, Piperidin- und Morpholinringe entstehen, die durch Methyl oder Phenyl substituiert sein können, beispielsweise 4-Methylpiperidinyl, 3,5-Dimethylpiperidinyl, 3,5-Dimethylmorpholinyl, 4-Phenylpiperidinyl, 3-Ethyl-4-methyl-piperidinyl, Morpholinyl, 2,6-Dimethylpiperidinyl, Pyrrolidinyl, 3,4-Dimethyl-pyrrolidinyl.

R und R' können zusammen mit dem Stickstoffatom, dessen Substituenten sie sind, auch einen Octahydroindol- oder Methanoazepanylrest bilden, der durch Methyl einfach oder mehrfach substituiert sein kann, z.B. den 2,3,3-Trimethyl-octahydroindolylrest oder den 2,6-Methano-4,4,6-trimethyl-R' in Formel I kann auch Wasserstoff oder Acetyl bedeuten, wenn R für einen gegebenenfalls durch Halogen, z.B. Chlor oder Brom, substituierten Phenylrest steht. Als Aminoreste $-N<^{R'}_{R}$ in Formel I kommen somit beispielsweise N-Acetyl-N-(4-chlor-phenyl)-amino oder N-(4-Chlorphenyl)-amino in Betracht.

Bevorzugte N-Benzoyl-N'-phenylharnstoffe der Formel I sind solche, bei denen X in o-Stellung zur Carbonylgruppe steht und Fluor oder Chlor, n 1 oder 2, Y Wasserstoff, Fluor, Chlor, Brom oder Trifluormethyl und Y' Wasserstoff bedeuten und R und R' zusammen eine gegebenenfalls durch Methyl oder Ethyl einfach oder zweifach substituierte Alkylenkette mit 4 oder 5 Kohlenstoffatomen bilden, oder solche, bei denen X in o-Stellung zur Carbonylgruppe steht und Fluor oder

Chlor, n 1 oder 2, Y Wasserstoff, Fluor, Chlor, Brom oder Trifluormethyl, Y' Wasserstoff, und R und R' unabhängig voneinander einen Alkylrest mit 2 bis 9 Kohlenstoffatomen bedeuten.

Man erhält die N-Benzoyl-N'-phenylharnstoffe der Formel I durch Umsetzung eines Benzoylisocyanats der Formel

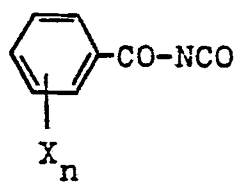
(II),

in der
X und n die oben genannten Bedeutungen haben,

mit einem Phenylendiamin der Formel

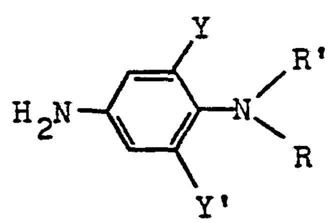
(III),

in der
Y, Y', R und R' die oben genannten Bedeutungen haben,

in Gegenwart eines inerten organischen Lösungsmittels bei einer Temperatur im Bereich zwischen 0 und 80°C.

Die Umsetzung wird in Gegenwart geeigneter Lösungs- und Verdünnungsmittel durchgeführt. Als solche kommen praktisch alle inerten organischen Solventien in Betracht. Hierzu gehören insbesondere aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan; cyclische und acyclische Ether,

wie Diethylether, Dibutylether, Tetrahydrofuran, Dioxan; acyclische und cyclische Ketone, wie Aceton, Methylethylketon, Methylisopropylketon, Cyclohexanon; Nitrile, wie mittel können verwendet werden.

Die Reaktionstemperatur kann innerhalb eines größeren Bereichs variiert werden. Sie liegt in der Regel zwischen 0 und 80°C. Die Umsetzung verläuft aufgrund der exothermen Reaktion üblicherweise bei einer Temperatur im Bereich zwischen 20 und 60°C.

Man läßt die Umsetzung im allgemeinen bei Normaldruck ablaufen. Sie kann diskontinuierlich durchgeführt werden.

Die Reaktionskomponenten werden vorzugsweise in äquimolarem Verhältnis eingesetzt. Ein Überschuß der einen oder anderen Komponente bringt keine wesentlichen Vorteile. Die Umsetzung verläuft praktisch quantitiativ.

Die N-Benzoyl-N'-phenyl-harnstoffe der Formel I fallen als Festprodukte an, die in der Regel analysenrein sind, andernfalls aber durch Umkristallisieren gereinigt werden können. Zu ihrer Charakterisierung dienen Elementaranalyse, Schmelzpunkt, IR- und NMR-Spektren.

Zweckmäßigerweise wird das substituierte Phenylendiamin der Formel III zusammen mit dem Lösungs- oder Verdünnungsmittel vorgelegt, dann wird das Isocyanat der Formel II zugegeben. Nach mehrstündiger Reaktionsdauer, in der Regel nach 2 Stunden, wird das Produkt dann abgesaugt und unter vermindertem Druck getrocknet.

Die Herstellung der Benzoylisocyanate der Formel II ist bekannt (J. Org. Chem. 28, 1805-1811 (1963)).

Die Phenylendiamine werden nach üblichen Methoden aus Nitranilinen durch Reduktion hergestellt (Weygand-Hilgetag, Organisch-Chemische Experimentierkunst, 570-577, (1970)). Die Nitraniline werden aus Halogennitrobenzolen und den geeigneten Aminen hergestellt (Weygand-Hilgetag, Organisch--Chemische Experimentierkunst, 475 (1970)).

Die Harnstoffe der Formel I können auch durch Umsetzung von Amiden der Formel

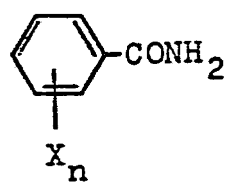

(IV),

mit Isocyanaten der Formel

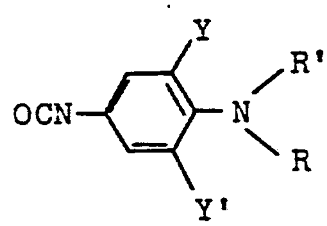

(V),

hergestellt werden. In den Formeln IV und V haben X, n, Y, Y', R' und R die oben genannten Bedeutungen.

Die Reaktionspartner werden hierbei in äquimolaren Mengen gegebenenfalls in Gegenwart eines inerten organischen Lösungsmittels umgesetzt. Die Reaktionstemperatur liegt zwischen 0 und 140°C, vorzugsweise zwischen 60 und 100°C. Gegebenenfalls kann ein Katalysator, wie Triethylamin, zugesetzt werden. Als Lösungsmittel kommen die gleichen Solventien in Betracht, die bei der Umsetzung der Verbindungen der Formel II mit den Verbindungen der Formel III verwendet werden können.

Beispiel 1

a) 50,9 Teile 3,5-Dimethylpiperidin, 300 Teile Sulfolan, 62,1 Teile $K_2CO_3$ und 86,4 Teile 3,4-Dichlornitrobenzol werden 1,5 Stunden bei 140 bis 150°C gerührt. Bei Raumtemperatur wird in 1200 Teilen Wasser eingerührt. Der Rückstand wird mit 500 Teilen 70 % Ethanol gewaschen und abgesaugt. Nach dem Trocknen erhält man 92,6 Teile 2-Chlor-4-nitro-4-(3',5'-dimethylpiperidyl)-benzol vom Fp. 58-64°C.

b) 91 Teile 2-Chlor-4-nitro-4-(3',5'-dimethylpiperidyl)-benzol werden in 1500 Teilen Tetrahydrofuran mit 15 Teilen Raney-Nickel versetzt. Dann werden 200 bar Wasserstoff aufgepreßt, und es wird 15 Stunden bei 50°C gehalten. Nachdem abfiltriert und einrotiert wurde, erhält man 81,5 Teile 3-Chlor-4-(3',5'-dimethylpiperidyl)-anilin als zähes Öl.

c) 7,2 Teile 3-Chlor-4-(3',5'-dimethylpiperidyl)-anilin werden in 100 Teilen Toluol gelöst und mit 6 Teilen 2,6-Difluorbenzoylisocyanat versetzt. Man rührt über Nacht bei Raumtemperatur, saugt ab und trocknet. Man erhält 11,2 Teile N-(2,6-Difluorbenzoyl)-N'-[3-chlor-4-(3',5'-dimethylpiperidyl)-phenyl]-harnstoff vom Fp. 177-181°C.

Beispiel 2

6,0 Teile 3-Chlor-4-(3',5'-dimethylpiperidyl)-anilin werden in 60 Teilen Toluol gelöst und tropfenweise mit 5,8 Teilen 2,6-Dichlorbenzoylisocyanat versetzt. Die Temperatur steigt dabei um 20°C. Über Nacht wird bei Raumtemperatur gerührt. Der gebildete Niederschlag wird abgesaugt und getrocknet.

Man erhält 5,1 Teile N-(2,6-Dichlorbenzoyl)-N'-[3-chlor-4-(3',5'-dimethylpiperidyl)-phenyl]-harnstoff vom Fp. 223-225°C.

Analog werden die folgenden N-Benzoyl-N'-phenylharnstoffe der Formel I erhalten:

| Nr. | Verbindung | Fp. [°C] |
|---|---|---|
| 1 | N-(2,6-Difluorbenzoyl)-N'-(3-fluor-4-piperidylphenyl)-harnstoff | 227-229 |
| 2 | N-(2,6-Dichlorbenzoyl)-N'-(3-fluor-4-piperidylphenyl)-harnstoff | 249-252 |
| 3 | N-(2-Chlorbenzoyl)-N'-(3-fluor-4-piperidylphenyl)-harnstoff | 218-221 |
| 4 | N-(2-Chlor-6-fluorbenzoyl)-N'-(3-fluor-4-piperidylphenyl)-harnstoff | |
| 5 | N-(2,6-Difluorbenzoyl)-N'-[3-fluor-4-(3',5'-dimethylpiperidyl)-phenyl]-harnstoff | 185-187 |
| 6 | N-(2,6-Dichlorbenzoyl)-N'-[3-fluor-4-(3',5'-dimethylpiperidyl)-phenyl]-harnstoff | 208-214 |
| 7 | N-(2-Chlorbenzoyl)-N'-(3-fluor-4-(3',5'-dimethyliperidyl)-phenyl)-harnstoff | 176-180 |
| 8 | N-(2-Chlor-6-fluorbenzoyl)-N'-[3-fluor-4-(3',5'-dimethylpiperidyl)-phenyl]-harnstoff | 172-175 |
| 9 | N-(2,6-Difluorbenzoyl)-N'-[3-fluor-4-(3',5'-dimethylmorpholino)-phenyl]-harnstoff | 185-187 |
| 10 | N-(2,6-Dichlorbenzoyl)-N'-[3-fluor-4-(3',5'-dimethylmorpholino)-phenyl]-harnstoff | |
| 11 | N-(2-Chlorbenzoyl)-N'-[3-fluor-4-(3',5'-dimethylmorpholinophenyl)-harnstoff | |
| 12 | N-(2-Chlor-6-fluorbenzoyl)-N'-[3-fluor-4-(3',5'-dimethylmorpholino)-phenyl]-harnstoff | |
| 13 | N-(2,6-Difluorbenzoyl)-N'-(3-fluor-4-morpholinyl-phenyl)-harnstoff | |
| 14 | N-(2,6-Dichlorbenzoyl)-N'-(3-fluor-4-morpholinyl-phenyl)-harnstoff | |
| 15 | N-(2-Chlorbenzoyl)-N'-(3-fluor-4-morpholinyl-phenyl)-harnstoff | |
| 16 | N-(2-Chlor-6-fluorbenzoyl)-N'-(3-fluor-4-morpholinyl-phenyl)-harnstoff | |

| Nr. | Verbindung | Fp. [°C] |
|---|---|---|
| 17 | N-(2,6-Difluorbenzoyl)-N'-[3-fluor-4-(di-1-methylpropylamino)-phenyl]-harnstoff | |
| 18 | N-(2,6-Dichlorbenzoyl)-N'-[3-fluor-4-(di-1-methylpropylamino)-phenyl]-harnstoff | |
| 19 | N-(2-Chlorbenzoyl)-N'-[3-fluor-4-(di-1-methylpropylamino)-phenyl]-harnstoff | |
| 20 | N-(2-Chlor-6-fluorbenzoyl)-N'-[3-fluor-4-(di-1-methylpropylamino)-phenyl]-harnstoff | |
| 21 | N-(2,6-Difluorbenzoyl)-N'-(3-chlor-4-piperidylphenyl)-harnstoff | |
| 22 | N-(2,6-Dichlorbenzoyl)-N'-(3-chlor-4-piperidylphenyl)-harnstoff | |
| 23 | N-(2-Chlorbenzoyl)-N'-(3-chlor-4-piperidylphenyl)-harnstoff | |
| 24 | N-(2-Chlor-6-fluorbenzoyl)-N'-(3-chlor-4-piperidylphenyl)-harnstoff | |
| 25 | N-(2,6-Difluorbenzoyl)-N'-[3-chlor-4-(3',5'-dimethylpiperidyl)-phenyl]-harnstoff | 177-181 |
| 26 | N-(2,6-Dichlorbenzoyl)-N'-[3-chlor-4-(3',5'-dimethylpiperidyl)-phenyl]-harnstoff | 223-225 |
| 27 | N-(2-Chlorbenzoyl)-N'-[3-chlor-4-(3',5'-dimethylpiperidyl)-phenyl]-harnstoff | 192-196 |
| 28 | N-(2-Chlor-6-fluorbenzoyl)-N'-[3-chlor-4-(3',5'-dimethylpiperidyl-phenyl]-harnstoff | 172-180 |
| 29 | N-(2,6-Difluorbenzoyl)-N'-[3-chlor-4-(3',5'-dimethylmorpholinyl-phenyl]-harnstoff | 215-220 |
| 30 | N-(2,6-Dichlorbenzoyl)-N'-[3-chlor-4-(3',5'-dimethylmorpholinyl-phenyl]-harnstoff | |
| 31 | N-(2-Chlorbenzoyl)-N'-[3-chlor-4-(3',5'-dimethylmorpholinyl-phenyl]-harnstoff | |

| Nr. | Verbindung | Fp. [°C] |
|---|---|---|
| 32 | N-(2-Chlor-6-fluorbenzoyl)-N'-[3-chlor-4-(3',5'-dimethylmorpholinyl-phenyl]-harnstoff | |
| 33 | N-(2,6-Difluorbenzoyl)-N'-(3-chlor-4-morpholinyl-phenyl)-harnstoff | 235-237 |
| 34 | N-(2,6-Dichlorbenzoyl)-N'-(3-chlor-4-morpholinyl-phenyl)-harnstoff | |
| 35 | N-(2-Chlorbenzoyl)-N'-(3-chlor-4-morpholinyl-phenyl)-harnstoff | 225-229 |
| 36 | N-(2-Chlor-6-fluorbenzoyl)-N'-(3-chlor-4-morpholinyl-phenyl)-harnstoff | |
| 37 | N-(2,6-Difluorbenzoyl)-N'-[3-chlor-4-(di-sec.butylamino)-phenyl]-harnstoff | 159-167 |
| 38 | N-(2,6-Dichlorbenzoyl)-N'-[3-chlor-4-(di-1-methylpropylamino)-phenyl]-harnstoff | |
| 39 | N-(2-Chlorbenzoyl)-N'-[3-chlor-4-(di-1-methylpropylamino)-phenyl]-harnstoff | |
| 40 | N-(2-Chlor-6-fluorbenzoyl)-N'-[3-chlor-4-(di-1-methylpropylamino)-phenyl]-harnstoff | |
| 41 | N-(2,6-Difluorbenzoyl)-N'-(3-brom-4-piperidylphenyl)-harnstoff | 206-209 |
| 42 | N-(2,6-Dichlorbenzoyl)-N'-(3-brom-4-piperidylphenyl)-harnstoff | |
| 43 | N-(2-Chlorbenzoyl)-N'-(3-brom-4-piperidylphenyl)-harnstoff | 207-210 |
| 44 | N-(2-Chlor-6-fluorbenzoyl)-N'-(3-brom-4-piperidylphenyl)-harnstoff | |
| 45 | N-(2,6-Difluorbenzoyl)-N'-[3-brom-4-(3',5'-dimethylpiperidyl)-phenyl]-harnstoff | |
| 46 | N-(2,6-Dichlorbenzoyl)-N'-[3-brom-4-(3',5'-dimethylpiperidyl)-phenyl]-harnstoff | |
| 47 | N-(2-Chlorbenzoyl)-N'-[3-brom-4-(3',5'-dimethylpiperidyl)-phenyl]-harnstoff | |
| 48 | N-(2-Chlor-6-fluorbenzoyl)-N'-[3-brom-4-(3',5'-dimethylpiperidyl)-phenyl]-harnstoff | |

| Nr. | Verbindung | Fp. [°C] |
|---|---|---|
| 49 | N-(2,6-Difluorbenzoyl)-N'-[3-brom-4-(3',5'-dimethylmorpholinyl-phenyl]-harnstoff | |
| 50 | N-(2,6-Dichlorbenzoyl)-N'-[3-brom-4-(3',5'-dimethylmorpholinyl-phenyl]-harnstoff | |
| 51 | N-(2-Chlorbenzoyl)-N'-[3-brom-4-(3',5'-dimethylmorpholinyl-phenyl]-harnstoff | |
| 52 | N-(2-Chlor-6-fluorbenzoyl)-N'-[3-brom-4-(3',5'-dimethylmorpholinyl-phenyl]-harnstoff | |
| 53 | N-(2,6-Difluorbenzoyl)-N'-(3-brom-4-morpholinyl-phenyl)-harnstoff | |
| 54 | N-(2,6-Dichlorbenzoyl)-N'-(3-brom-4-morpholinyl-phenyl)-harnstoff | |
| 55 | N-(2-Chlorbenzoyl)-N'-(3-brom-4-morpholinyl-phenyl)-harnstoff | |
| 56 | N-(2-Chlor-6-fluorbenzoyl)-N'-(3-brom-4-morpholinyl-phenyl)-harnstoff | |
| 57 | N-(2,6-Difluorbenzoyl)-N'-[3-brom-4-(di-1-methylpropylamino)-phenyl]-harnstoff | |
| 58 | N-(2,6-Dichlorbenzoyl)-N'-[3-brom-4-(di-1-methylpropylamino)-phenyl]-harnstoff | |
| 59 | N-(2-Chlorbenzoyl)-N'-[3-brom-4-(di-1-methylpropylamino)-phenyl]-harnstoff | |
| 60 | N-(2-Chlor-6-fluorbenzoyl)-N'-[3-brom-4-(di-1-methylpropylamino)-phenyl]-harnstoff | |
| 61 | N-(2,6-Difluorbenzoyl)-N'-(4-piperidylphenyl)-harnstoff | |
| 62 | N-(2,6-Dichlorbenzoyl)-N'-(4-piperidylphenyl)-harnstoff | |
| 63 | N-(2-Chlorbenzoyl)-N'-(4-piperidylphenyl)-harnstoff | |
| 64 | N-(2-Chlor-6-fluorbenzoyl)-N'-(4-piperidylphenyl)-harnstoff | |
| 65 | N-(2,6-Difluorbenzoyl)-N'-[4-(3',5'-dimethylpiperidyl)-phenyl]-harnstoff | 215-220 |

| Nr. | Verbindung | Fp. [°C] |
|---|---|---|
| 66 | N-(2,6-Dichlorbenzoyl)-N'-[4-(3',5'-dimethylpiperidyl)-phenyl]-harnstoff | 184-188 |
| 67 | N-(2-Chlorbenzoyl)-N'-[4-(3',5'-dimethylpiperidyl)-phenyl]-harnstoff | 179-181 |
| 68 | N-(2-Chlor-6-fluorbenzoyl)-N'-[4-(3',5'-dimethylpierpidyl)-phenyl]-harnstoff | |
| 69 | N-(2,6-Difluorbenzoyl)-N'-[4-(3',5'-dimethylmorpholinyl-phenyl]-harnstoff | |
| 70 | N-(2,6-Dichlorbenzoyl)-N'-[4-(3',5'-dimethylmorpholinyl-phenyl]-harnstoff | |
| 71 | N-(2-Chlorbenzoyl)-N'-[4-(3',5'-dimethylmorpholinyl-phenyl]-harnstoff | |
| 72 | N-(2-Chlor-6-fluorbenzoyl)-N'-[4-(3',5'-dimethylmorpholinyl-phenyl]-harnstoff | |
| 73 | N-(2,6-Difluorbenzoyl)-N'-(4-morpholinyl-phenyl)-harnstoff | |
| 74 | N-(2,6-Dichlorbenzoyl)-N'-(4-morpholinyl-phenyl)-harnstoff | |
| 75 | N-(2-Chlorbenzoyl)-N'-(4-morpholinyl-phenyl)-harnstoff | |
| 76 | N-(2-Chlor-6-fluorbenzoyl)-N'-(4-morpholinyl-phenyl)-harnstoff | |
| 77 | N-(2,6-Difluorbenzoyl)-N'-[4-(di-sec.butylamino)-phenyl]-harnstoff | |
| 78 | N-(2,6-Dichlorbenzoyl)-N'-[4-(di-sec.butylamino)-phenyl]-harnstoff | |
| 79 | N-(2-Chlorbenzoyl)-N'-[4-(di-sec.butylamino)-phenyl]-harnstoff | |
| 80 | N-(2-Chlor-6-fluorbenzoyl)-N'-[4-(di-1-methylpropylamino)-phenyl]-harnstoff | |
| 81 | N-(2,6-Difluorbenzoyl)-N'-[3-chlor-4-(3'-ethyl-4'-methyl-piperidyl)-phenyl]-harnstoff | 210-211 |
| 82 | N-(2,6-Dichlorbenzoyl)-N'-[3-chlor-4-(3'-ethyl-4'-methyl-piperidyl)-phenyl]-harnstoff | 193-195 |

| Nr. | Verbindung | Fp. [°C] |
|---|---|---|
| 83 | N-(2-Chlorbenzoyl)-N'-[3-chlor-4-(3'-ethyl-4'-methyl-piperidyl)-phenyl]-harnstoff | 190-191 |
| 84 | N-(2-Chlor-6-fluorbenzoyl)-N'-[3-chlor-4-(3'-ethyl-4'-methyl-piperidyl)-phenyl]-harnstoff | |
| 85 | N-(2,6-Difluorbenzoyl)-N'-[3-chlor-4-(2,6-methano-4,4,6-trimethyl-azepan-(1)-yl)-phenyl]-harnstoff | 218-220 |
| 86 | N-(2,6-Dichlorbenzoyl)-N'-[3-chlor-4-(2,6-methano-4,4,6-trimethyl-azepan-(1)-yl)-phenyl]-harnstoff | 230-232 |
| 87 | N-(2-Chlorbenzoyl)-N'-[3-chlor-4-(2,6-methano-4,4,6-trimethylazepan-(1)-yl)-phenyl]-harnstoff | 201-203 |
| 88 | N-(2-Chlor-6-fluorbenzoyl)-N'-[3-chlor-4-(2,6-methano-4,4,6-trimethyl-azepan-(1)-yl)-phenyl]-harnstoff | |
| 89 | N-(2,6-Difluorbenzoyl)-N'-[3-chlor-4-(2',3',3'-trimethyl-octahydroindol-(1)-yl)-phenyl]-harnstoff | 220-222 |
| 90 | N-(2,6-Dichlorbenzoyl)-N'-[3-chlor-4-(2',3',3'-trimethyl-octahydroindol-(1)-yl)-phenyl]-harnstoff | 201-203 |
| 91 | N-(2-Chlorbenzoyl)-N'-[3-chlor-4-(2',3',3'-trimethyl-octahydroindol-(1)-yl)-phenyl]-harnstoff | 201-202 |
| 92 | N-(2-Chlor-6-fluorbenzoyl)-N'-[3-chlor-4-(2',3',3'-trimethyl-octahydro-indol-(1)-yl)-phenyl]-harnstoff | |
| 93 | N-(2,6-Difluorbenzoyl)-N'-[3-chlor-4-(N"-methyl-N"-benzylamino)-phenyl]-harnstoff | 190-192 |
| 94 | N-(2,6-Dichlorbenzoyl)-N'-[3-chlor-4-(N"-methyl-N"-benzylamino)-phenyl]-harnstoff | 190-192 |
| 95 | N-(2-Chlorbenzoyl)-N'-[3-chlor-4-(N"-methyl-N"-benzylamino)-phenyl]-harnstoff | 189-190 |

| Nr. | Verbindung | Fp. [°C] |
|---|---|---|
| 96 | N-(2-Chlor-6-fluorbenzoyl)-N'-[3-chlor-4-(N"-methyl-N"-benzylamino)--phenyl]-harnstoff | |
| 97 | N-(2,6-Difluorbenzoyl)-N'-[3-chlor-4-(4'-chlorphenylamino)-phenyl]-harnstoff | 209-211 |
| 98 | N-(2,6-Dichlorbenzoyl)-N'-[3-chlor-4-(4'-chlorphenylamino)-phenyl]-harnstoff | 186-189 |
| 99 | N-(2-Chlorbenzoyl)-N'-[3-chlor-4-(4'-chlorphenylamino)-phenyl]-harnstoff | 183-185 |
| 100 | N-(2-Chlor-6-fluorbenzoyl)-N'-[3-chlor-4-(4'-chlorphenylamino)-phenyl]--harnstoff | |
| 101 | N-(2,6-Difluorbenzoyl)-N'-[3-chlor-4-(N"-acetyl-N"-4-chlorphenyl)-aminophenyl]-harnstoff | 188-191 |
| 102 | N-(2,6-Dichlorbenzoyl)-N'-[3-chlor-4-(N"-acetyl-N"-4-chlorphenyl)-aminophenyl]-harnstoff | |
| 103 | N-(2-Chlorbenzoyl)-N'-[3-chlor-4-(N"-acetyl-N"-4-chlorphenyl)-aminophenyl]-harnstoff | 190-195 |
| 104 | N-(2-Chlor-6-fluorbenzoyl)-N'-[3-chlor-4-(N"-acetyl-N"-4-chlorphenyl)--aminophenyl]-harnstoff | |
| 105 | N-(2,6-Difluorbenzoyl)-N'-[3-chlor-4-(4'-phenylpiperidyl)-phenyl]--harnstoff | 254-256 |
| 106 | N-(2,6-Dichlorbenzoyl)-N'-[3-chlor-4-(4'-phenylpiperidyl)-phenyl]--harnstoff | 238-240 |
| 107 | N-(2-Chlorbenzoyl)-N'-[3-chlor-4-(4'-phenylpiperidyl)-phenyl]-harnstoff | 237-240 |
| 108 | N-(2-Chlor-6-fluorbenzoyl)-N'-[3-chlor-4-(4'-phenylpiperidyl)-phenyl]--harnstoff | |

| Nr. | Verbindung | Fp. [°C] |
|---|---|---|
| 109 | N-(2,6-Difluorbenzoyl)-N'-[3-chlor-4-(N",N"-didodecyl)-aminophenyl]-harnstoff | Paste |
| 110 | N-(2,6-Dichlorbenzoyl)-N'-[3-chlor-4-(N",N"-didodecyl)-aminophenyl]-harnstoff | Paste |
| 111 | N-(2-Chlorbenzoyl)-N'-[3-chlor-4-(N",N"-didodecyl)-aminophenyl]-harnstoff | Paste |
| 112 | N-(2-Chlor-6-fluorbenzoyl)-N'-[3-chlor-4-(N",N"-didodecyl)-aminophenyl]-harnstoff | |
| 113 | N-(2,6-Difluorbenzoyl)-N'-(3,5-dichlor-4-piperidyl-phenyl)-harnstoff | |
| 114 | N-(2,6-Dichlorbenzoyl)-N'-(3,5-dichlor-4-piperidyl-phenyl)-harnstoff | |
| 115 | N-(2-Chlorbenzoyl)-N'-(3,5-dichlor-4-piperidyl-phenyl)-harnstoff | |
| 116 | N-(2-Chlor-6-fluor-benzoyl)-N'-(3,5-dichlor-4-piperidyl-phenyl)-harnstoff | |
| 117 | N-(2,6-Difluorbenzoyl)-N'-(3,5-dichlor-4-pyrrolidinyl-phenyl)-harnstoff | 231-235 |
| 118 | N-(2,6-Dichlorbenzoyl)-N'-(3,5-dichlor-4-pyrrolidinyl-phenyl)-harnstoff | 252-259 |
| 119 | N-(2-Chlorbenzoyl)-N'-(3,5-dichlor-4-pyrrolidinyl-phenyl)-harnstoff | 195-203 |
| 120 | N-(2-Chlor-6-fluorbenzoyl)-N'-(3,5-dichlor-4-pyrrolidinyl-phenyl)-harnstoff | |
| 121 | N-(2,6-Difluorbenzoyl)-N'-(3,5-dibrom-4-piperidyl-phenyl)-harnstoff | 226-230 |
| 122 | N-(2,6-Dichlorbenzoyl)-N'-(3,5-dibrom-4-piperidyl-phenyl)-harnstoff | 271-274 |
| 123 | N-(2-Chlorbenzoyl)-N'-(3,5-dibrom-4-piperidyl-phenyl)-harnstoff | 219-223 |
| 124 | N-(2-Chlor-6-fluorbenzoyl)-N'-(3,5-dibrom-4-piperidyl-phenyl)-harnstoff | |
| 125 | N-(2,6-Difluorbenzoyl)-N'-(3,5-dibrom-4-pyrrolidinyl-phenyl)-harnstoff | 244-246 |
| 126 | N-(2,6-Dichlorbenzoyl)-N'-(3,5-dibrom-4-pyrrolidinyl-phenyl)-harnstoff | 261-262 |
| 127 | N-(2-Chlorbenzoyl)-N'-(3,5-dibrom-4-pyrrolidinyl-phenyl)-harnstoff | 235-236 |

| Nr. | Verbindung | Fp. [°C] |
|---|---|---|
| 128 | N-(2-Chlor-6-fluorbenzoyl)-N'-(3,5-dibrom-4-pyrrolidinyl-phenyl)-harnstoff | |
| 129 | N-(2,6-Difluorbenzoyl)-N'-(3-methyl-4-piperidyl-phenyl)-harnstoff | 193-196 |
| 130 | N-(2,6-Dichlorbenzoyl)-N'-(3-methyl-4-piperidyl-phenyl)-harnstoff | 119-122 |
| 131 | N-(2-Chlorbenzoyl)-N'-(3-methyl-4-piperidyl-phenyl)-harnstoff | 172-175 |
| 132 | N-(2-Chlor-6-fluorbenzoyl)-N'-(3-methyl-4-piperidyl-phenyl)-harnstoff | |
| 133 | N-(2,6-Difluorbenzoyl)-N'-[3-methyl-4-(3,5-dimethylpiperidyl)-phenyl]-harnstoff | |
| 134 | N-(2,6-Dichlorbenzoyl)-N'-[3-methyl-4-(3,5-dimethylpiperidyl)-phenyl]-harnstoff | |
| 135 | N-(2-Chlorbenzoyl)-N'-[3-methyl-4-(3,5-dimethylpiperidyl)-phenyl]-harnstoff | |
| 136 | N-(2-Chlor-6-fluorbenzoyl)-N'-[3-methyl-4-(3,5-dimethylpiperidyl)-phenyl]-harnstoff | |
| 137 | N-(2,6-Difluorbenzoyl)-N'-(3-methyl-4-pyrrolidinyl-phenyl)-harnstoff | |
| 138 | N-(2,6-Dichlorbenzoyl)-N'-(3-methyl-4-pyrrolidinyl-phenyl)-harnstoff | |
| 139 | N-(2-Chlorbenzoyl)-N'-(3-methyl-4-pyrrolidinyl-phenyl)-harnstoff | |
| 140 | N-(2-Chlor-6-fluorbenzoyl)-N'-(3-methyl-4-pyrrolidinyl-phenyl)-harnstoff | |
| 141 | N-(2,6-Difluorbenzoyl)-N'-(3-trifluormethyl-4-piperidyl-phenyl)-harnstoff | 235-240 |
| 142 | N-(2,6-Dichlorbenzoyl)-N'-(3-trifluormethyl-4-piperidyl-phenyl)-harnstoff | 189-192 |
| 143 | N-(2-Chlorbenzoyl)-N'-(3-trifluormethyl-4-piperidyl-phenyl)-harnstoff | 192-197 |
| 144 | N-(2-Chlor-6-fluorbenzoyl)-N'-(3-trifluormethyl-4-piperidyl-phenyl)-harnstoff | |

| Nr. | Verbindung | Fp. [°C] |
|---|---|---|
| 145 | N-(2,6-Difluorbenzoyl)-N'-[3-trifluormethyl-4-(3,5-dimethylpiperidyl)-phenyl]-harnstoff | |
| 146 | N-(2,6-Dichlorbenzoyl)-N'-[3-trifluormethyl-4-(3,5-dimethylpiperidyl)-phenyl]-harnstoff | |
| 147 | N-(2-Chlorbenzoyl)-N'-[3-trifluormethyl-4-(3,5-dimethylpiperidyl)-phenyl]-harnstoff | |
| 148 | N-(2-Chlor-6-fluorbenzoyl)-N'-[3-trifluormethyl-4-(3,5-dimethylpiperidyl)-phenyl]-harnstoff | |
| 149 | N-(2,6-Difluorbenzoyl)-N'-[3-trifluormethyl-4-pyrrolidinyl-phenyl)-harnstoff | |
| 150 | N-(2,6-Dichlorbenzoyl)-N'-(3-trifluormethyl-4-pyrrolidinyl-phenyl)-harnstoff | |
| 151 | N-(2-Chlorbenzoyl)-N'-(3-trifluormethyl-4-pyrrolidinyl-phenyl)-harnstoff | 190-192 |
| 152 | N-(2-Chlor-6-fluorbenzoyl)-N'-(3-trifluormethyl-4-pyrrolidinyl-phenyl)-harnstoff | |
| 153 | N-(2,5-Difluorbenzoyl)-N'-(3-chlor-4-pyrrolidinyl-phenyl)-harnstoff | 196-200 |
| 154 | N-(2,6-Difluorbenzoyl)-N'-(3-chlor-4-pyrrolidinyl-phenyl)-harnstoff | 244-247 |
| 155 | N-(2-Chlorbenzoyl)-N'-(3-chlor-4-pyrrolidinyl-phenyl)-harnstoff | 190-192 |
| 156 | N-(2,6-Difluorbenzoyl)-N'-(3-brom-4-pyrrolidinyl-phenyl)-harnstoff | 201-203 |
| 157 | N-(2,6-Dichlorbenzoyl)-N'-(3-brom-4-pyrrolidinyl-phenyl)-harnstoff | 231-235 |
| 158 | N-(2,6-Difluorbenzoyl)-N'-(3-fluor-4-di-2-methylpropylamino-phenyl)-harnstoff | |
| 159 | N-(2,6-Dichlorbenzoyl)-N'-(3-fluor-4-di-2-methylpropylamino-phenyl)-harnstoff | |
| 160 | N-(2-Chlorbenzoyl)-N'-(3-fluor-4-di-2-methylpropylamino-phenyl)-harnstoff | |
| 161 | N-(2-Fluor-6-chlorbenzoyl)-N'-(3-fluor-4-di-2-methylpropylamino-phenyl)-harnstoff | |

| Nr. | Verbindung | Fp. [°C] |
|---|---|---|
| 162 | N-(2,6-Difluorbenzoyl)-N'-(3-chlor-4-di-2-methylpropylamino-phenyl)-harnstoff | |
| 163 | N-(2,6-Dichlorbenzoyl)-N'-(3-chlor-4-di-2-methylpropylamino-phenyl)--harnstoff | |
| 164 | N-(2-Chlorbenzoyl)-N'-(3-chlor-4-di-2-methylpropylamino-phenyl)-harnstoff | |
| 165 | N-(2,6-Dichlorbenzoyl)-N'-[3-chlor-4-di-(1,3-dimethylbutylamino)-phenyl]--harnstoff | |
| 166 | N-(2,6-Difluorbenzoyl)-N'-[3-chlor-4-di-(1,3-dimethylbutylamino)-phenyl]--harnstoff | |
| 167 | N-(2-Chlorbenzoyl)-N'-[3-chlor-4-di-(1,3-dimethylbutylamino)-phenyl]--harnstoff | |
| 168 | N-(2,6-Difluorbenzoyl)-N'-[3-fluor-4-di-(1,3-dimethylbutylamino)-phenyl]--harnstoff | |
| 169 | N-(2,6-Dichlorbenzoyl)-N'-[3-fluor-4-di-(1,3-dimethylbutylamino)-phenyl]--harnstoff | |
| 170 | N-(2-Chlorbenzoyl)-N'-[3-fluor-4-di-(1,3-dimethylbutylamino)-phenyl]--harnstoff | |
| 171 | N-(2,6-Difluorbenzoyl)-N'-[3-chlor-4-(ethyl-cyclohexylamino)-phenyl]--harnstoff | |
| 172 | N-(2,6-Dichlorbenzoyl)-N'-[3-chlor-4-(ethyl-cyclohexylamino)-phenyl]--harnstoff | |
| 173 | N-(2-Chlorbenzoyl)-N'-[3-chlor-4-(ethyl-cyclohexylamino)-phenyl]-harnstoff | |

Die N-Benzoyl-N'-phenylharnstoffe der Formel I sind geeignet, Schädlinge aus der Klasse der Insekten und Spinnentiere wirksam zu bekämpfen. Sie sind geeignete Adultizide und Ovizide und können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm),

Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgs-

heuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Spinnentiere (Acarina), beispielsweise Ixodes ricinus (Holzbock), Ornithodorus moubata, Amblyomma americanum, Dermacentor silvarum, Boophilus microplus, Tetranychus telarius, Tetranychus atalnticus, Tetranychus pacificus, Paratetranychus pilosus, Bryobia praetiosa.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 3 Gewichtsteile der Verbindung Nr. 9 werden mit 97 Gewichtsteilen feinteiligem Kaolin innig vermischt. Man erhält auf diese Weise ein Stäubemittel, das 3 Gew.% des Wirkstoffs enthält.

II. 30 Gewichtsteile der Verbindung Nr. 25 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 29 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 82 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 28 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer

Sulfit-Ablauge und 7 Gewichtsteilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakteri-

zide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxy]-thio-acetimidat, Methyl-N',N'-dimethyl-N-[(methylcarbamoyl)oxy]-1-thiooxamidat, N-(2-Methyl-chlor-phenyl)-N',N'-dimethylformamidin, Tetrachlorthiophen, 1-(2,6-Difluor-benzoyl)-3-(4-chlor-phenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[p-methylsulfinyl-phenyl]-phosphorthioat,

O-Ethyl-S-phenyl-ethyl-phosphonodithioat, O,O-Diethyl-[2-chlor-1-(2,4-dichlorphenyl)-vinyl]-phosphat, O,O-Dimethyl-[-2-chlor-1-(2,4,5-trichlorphenyl)]-vinyl-phosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methyl-carbamoyl-methyl)-phosphorthioat, O,O-Dimethyl-S-(N-methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethyl-thio-methyl)-phosphordithioat, O,O-Diethyl-S-[(p-chlor-phenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorththioat, O,O-Diethyl-thiophosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]phosphorthioat, O,O-Diethyl-O-(2-pyrazinyl)-phosphorthioat, O,O-Diethyl-O-[2-isopropyl-4-methyl-pyrimidinyl(6)]-phosphorthioat,

O,O-Diethyl-O-[2-(diethylamino)-6-methyl-4pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amido-thioat, O,S-Dimethyl-N-acetyl-phosphoramidothioat, γ-Hexachlorcyclohexan, 1,1-Di-(p-methoxyphenyl)-2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,trans-chrysanthemat, 5-Benzyl-furyl-(3)-methyl-DL-cis,trans-chrysanthemat, 3-Phenoxybenzyl(±)-cis,trans-2,2-dimethyl-3-(2,2-dichlorvinyl)-cyclopropancarboxylat, α-Cyano-3-phenoxybenzyl(±)-cis,trans-2,2-dimethyl-3-(2,2-dichlorvinyl)-cyclopropancarboxylat, (s)-α-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,trans-chrysanthemat, 2-Methyl-5-(2-propinyl)-3-furylmethyl-chrysanthemat, (α-Cyano-3-phenoxybenzyl)-α-isopropyl-4-chlorphenylacetat.

In den nachstehenden Anwendungsbeispielen wurden als Vergleichsmittel die folgenden Verbindungen eingesetzt:

I

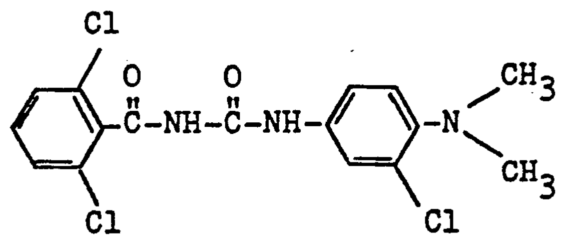

(aus J. Agr. Food Chem. 21, 353 (1973))

II

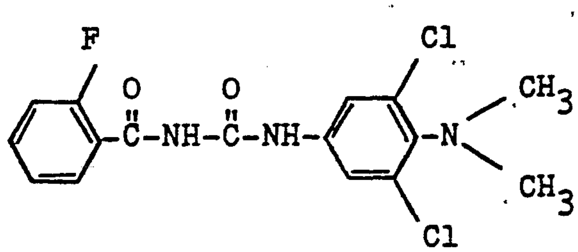

III

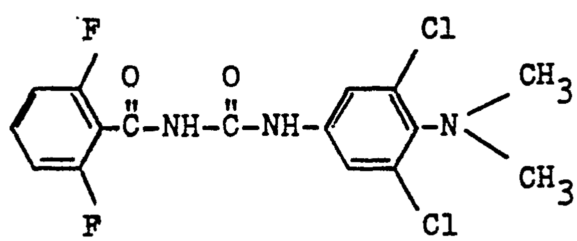

IV

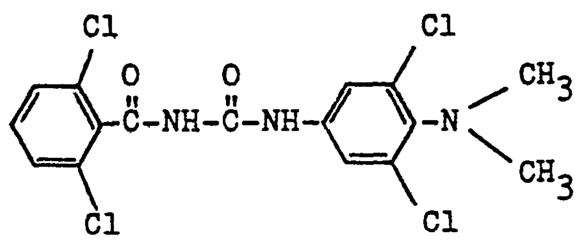

(II, III, IV aus EP 16729)

Besonders hervorzuheben ist die Hemmwirkung auf Blattläuse. So konnte bei Aphis fabae durch 0,005 % der Probe 26 die Koloniebildung total unterdrückt werden. Ein Effekt, der von dieser Substanzklasse bisher nicht bekannt ist.

Anwendungsbeispiel I

Zuchtversuch mit Moskito-Larven (Aedes aegypti)

200 ml Leitungswasser werden mit der Wirkstoffaufbereitung versetzt und darauf mit 20 bis 30 Moskito-Larven im 3. bis 4. Larvenstadium besetzt.

Die Gefäße stehen bei 25°C. Beobachtet werden Verpuppung und Schlüpfen der Imagines, welches nach 10 bis 12 Tagen erfolgt. Während der Beobachtungszeit wird einmal mit gepulvertem Zierfischfutter gefüttert.

Ergebnis:

| | | | | |
|---|---|---|---|---|
| Probe Nr. | 1 | 0,4 | ppm | 100 % Mort. |
| " | 5 | 0,002 | " | 100 % " |
| " | 6 | 0,01 | " | 100 % " |
| " | 8 | 0,01 | " | 100 % " |
| " | 25 | 0,04 | " | 100 % " |
| " | 28 | 0,004 | " | 100 % " |
| " | 121 | 0,001 | " | 100 % " |
| " | 125 | 0,02 | " | 100 % " |
| " | 129 | 0,04 | " | 100 % " |
| Vergleichsmittel | I | 1,0 | " | 60 % " |
| " | II | 1,0 | " | 60 % " |
| " | III | 0,1 | " | 100 % " |
| | | 0,04 | " | 60 % " |
| " | IV | 0,4 | " | 100 % " |
| | | 0,2 | " | 60 % " |

Anwendungsbeispiel II

Zuchtversuch mit Maiszünsler-Larven (Ostrinia nubilalis)

Die Zucht erfolgt auf einem Nährboden folgender Zusammensetzung:

| | |
|---|---|
| 515 g | Maismehl |
| 130 g | Weizenkeime |
| 137 g | Bierhefe |
| 18 g | Ascorbinsäure |
| 10 g | Cellulosepulver |
| 5 g | Nipagin |
| 20 g | Wessons Salz |
| 20 ml | Vitamonlösung |
| 80 g | Agar |
| 3100 ml | Wasser |

Je 50 ml füllt man in 100 ml Plastikbecher und mischt die wäßrige Wirkstoffaufbereitung sorgfältig unter. Nach Erkalten belegt man jedes Gefäß mit 4 Raupen (L 3). Pro Konzentration werden 5 Gefäße angesetzt.

Die Beobachtung erstreckt sich bis zum Schlüpfen der Falter.

Ergebnis:

| | | | | |
|---|---|---|---|---|
| Probe Nr. | 5 | 2,0 ppm | 85 % Mort. | |
| " | 25 | 1,0 " | 95 % " | |
| " | 26 | 1,0 " | 87 % " | |
| " | 37 | 0,4 " | 95 % " | |
| " | 117 | 2,0 " | 85 % " | |
| Vergleichsmittel | I | 40 " | 40 % " | |
| " | II | 40 " | 40 % " | |
| " | III | 4 " | 80 % " | |
| " | IV | 10 " | 85 % " | |

Anwendungsbeispiel III

Zuchtversuch mit Baumwollwanzen (Dysdercus intermedius)

Baumwollwanzen (Dysdercus intermedius) werden im 4. Larvenstadium in Petrischalen (Ø 10 cm) dem Wirkstoffbelag der Testsubstanz für 24 Stunden ausgesetzt.

Die Überlebenden züchtet man in 1 l-Gläsern auf feuchtem Quarzsand, der vorher mit der Wirkstofflösung versetzt wurde bis zum Schlüpfen der $F_1$-Generation.

Dabei entsprechen in der Behandlung

| | |
|---|---|
| 2,5 mg/Schale | 25 ppm im Sand |
| 1,0 mg/Schale | 10 ppm im Sand |
| 0,5 mg/Schale | 5 ppm im Sand |

usw.

Beurteilt wird Mortalität und Vermehrung.

Ergebnis:

| | | | |
|---|---|---|---|
| Probe Nr. | 28 | 2 ppm | keine $F_1$ |
| " | 117 | 2 " | " " |
| " | 119 | 2 " | " " |
| " | 123 | 1 " | " " |
| " | 125 | 2 " | " " |
| " | 127 | 2 " | " " |
| " | 129 | 4 " | " " |

Patentansprüche

1. N-Benzoyl-N'-phenylharnstoffe der Formel

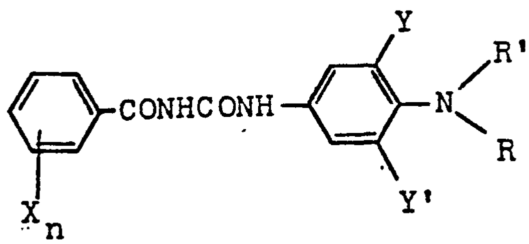

(I),

in der
X Chlor, Fluor, Brom oder Methyl,
n 0, 1, 2 oder 3,
Y und Y' unabhängig voneinander Wasserstoff, Fluor, Chlor, Brom, Methyl, Trifluormethyl und
R und R' unabhängig voneinander einen gegebenenfalls substituierten aliphatischen Kohlenwasserstoffrest mit 1 bis 15 Kohlenstoffatomen, wobei R und R' nicht gleichzeitig Methyl sein können, oder einen gegebenenfalls substituierten Phenylrest bedeuten oder zusammen eine gegebenenfalls durch Alkyl oder Phenyl einfach oder zweifach substituierte Alkylenkette mit 4 oder 5 Kohlenstoffatomen, die durch Sauerstoff unterbrochen sein kann, bilden oder zusammen mit dem Stickstoffatom, dessen Substituenten R und R' sind, einen gegebenenfalls durch Methyl einfach oder mehrfach substituierten Octahydroindolyl- oder Methanoazepanylrest bilden,
und R' auch Wasserstoff oder Acetyl bedeutet, wenn R für einen substituierten Phenylrest steht,
mit der Maßgabe, daß R und R' eine gegebenenfalls durch Alkyl oder Phenyl einfach oder zweifach substituierte Alkylenkette mit 4 oder 5 Kohlenstoffatomen, die durch Sauerstoff unterbrochen sein kann, bilden oder zusammen mit dem Stickstoffatom, dessen Substituenten R und R' sind, einen gegebenenfalls durch Methyl einfach oder mehrfach substituierten Octahydroindolyl- oder Methanoazepanylrest bilden, wenn Y und Y' jeweils Chlor oder Brom bedeuten.

2. N-Benzoyl-N'-phenylharnstoffe der Formel I gemäß Anspruch 1, in der X Fluor oder Chlor in o-Stellung zur Carbonylgruppe, n 1 oder 2, Y Wasserstoff, Fluor, Chlor, Brom oder Trifluormethyl und Y' Wasserstoff bedeuten und R und R' zusammen eine gegebenenfalls durch Methyl oder Ethyl einfach oder zweifach substituierte Alkylenkette mit 4 oder 5 Kohlenstoffatomen bilden.

3. N-Benzoyl-N'-phenylharnstoffe der Formel I gemäß Anspruch 1, in der X Fluor oder Chlor in o-Stellung zur Carbonylgruppe, n 1 oder 2, Y Wasserstoff, Fluor, Chlor, Brom oder Trifluormethyl, Y' Wasserstoff und R und R' unabhängig voneinander einen Alkylrest mit 2 bis 9 Kohlenstoffatomen bedeuten.

4. Verfahren zur Herstellung von N-Benzoyl-N'-phenylharnstoffen der Formel I gemäß Anspruch 1, dadurch gekennzeichnet, daß man ein Benzoylisocyanat der Formel

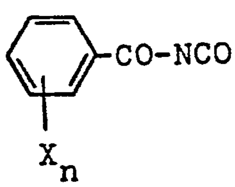

(II),

in der X und n die im Anspruch 1 genannten Bedeutungen haben,
mit einem Phenylendiamin der Formel

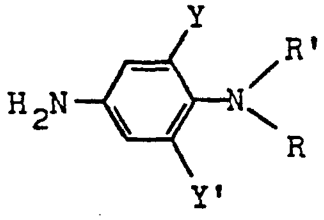

(III),

in der
Y, Y', R und R' die im Anspruch 1 genannten Bedeutungen haben,
in Gegenwart eines inerten organischen Lösungsmittels bei einer Temperatur im Bereich zwischen 0 und 80°C umsetzt.

5. Verfahren zur Herstellung von N-Benzoyl-N'-phenylharnstoffen der Formel I gemäß Anspruch 1, dadurch gekennzeichnet, daß man ein Amid der Formel

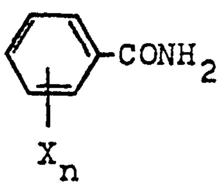

(IV),

in der
X und n die im Anspruch 1 genannten Bedeutungen haben,
mit einem Isocyanat der Formel

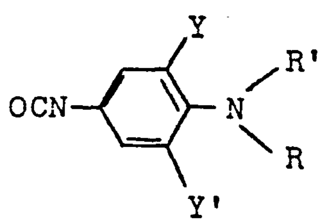

(V),

in der
Y, Y', R und R' die im Anspruch 1 genannten Bedeutungen haben,

in Gegenwart eines inerten organischen Lösungsmittels bei einer Temperatur zwischen 0 und 140°C umsetzt.

6. Insektizides und akarizides Mittel, enthaltend einen N-Benzoyl-N'-phenylharnstoff der Formel I gemäß Anspruch 1.

7. Insektizides und akarizides Mittel, enthaltend inerte Zusatzstoffe und einen N-Benzoyl-N'-phenylharnstoff der Formel I gemäß Anspruch 1.

8. Verwendung von N-Benzoyl-N'-phenylharnstoffen der Formel I gemäß Anspruch 1 zur Bekämpfung von Insekten und Spinnentieren.

9. Verfahren zur Bekämpfung von Insekten und Spinnentieren, <u>dadurch gekennzeichnet</u>, daß man eine wirksame Menge eines N-Benzoyl-N'-phenylharnstoffs der Formel I gemäß Anspruch 1 auf Insekten oder Spinnentiere und/oder deren Lebensraum einwirken läßt.

10. Verfahren zur Herstellung eines insektiziden und akariziden Mittels gemäß Anspruch 7, <u>dadurch gekennzeichnet</u>, daß man einen N-Benzoyl-N'-phenylharnstoff der Formel I gemäß Anspruch 1 mit oberflächenaktiven Verbindungen und/oder Streckmitteln und/oder Synergisten mischt.